# EUROPEAN PATENT APPLICATION

(11) **EP 1 306 076 A2**
(43) Date of publication of application: **02.05.2003**
(21) Application number: 02023439.9
(22) Date of filing: 19.10.2002
(51) Int. Cl.: A61K 7/06, A61K 7/48, A61K 9/20

(54) **Use of high-purity phenylsilsesquioxane liquids for the preparation of cosmetic and pharmaceutical compositions**

(30) Priority: 24.10.2001 US 2709
(71) Applicant: CLARIANT INTERNATIONAL LTD., 4132 Muttenz (CH)
(72) Inventor: Legrow, Gary E., Newberry, Florida 32669 (US); Terry, W. Leonard, Jr., Gainesville, Florida 32653 (US); Figueroa, Ray, Hollywood , Florida 33019 (US)
(74) Representative: Mikulecky, Klaus, Dr.

(57) **Abstract**

The invention relates to the use of phenylsilsesquioxane liquids of the general composition

Me₃SiO-[Si(OSiMe₃)(Ph)O]ₓ-SiMe₃

wherein Me is methyl, Ph is phenyl and x is an integer between 1 and 8, and wherein the phenylsilsesquioxanes are substantially free from alkoxysilanes, chlorosilanes, silanols, hexamethyldisiloxanes, organic compounds and inorganic compounds, for the preparation of cosmetic and pharmaceutical compositions.

## Description

### FIELD OF THE INVENTION

The present invention relates to the use of high-purity phenylsilsesquioxane liquids for the preparation of cosmetic and pharmaceutical compositions.

### BACKGROUND OF THE INVENTION

The use of phenyltrimethicones in cosmetic products is described in a large number of patents (UNITED States Patent 5,531,986, World Patent WO 2000038621, German Patent 19856852, and European Patent EP 943323).

Phenyltrimethicones are circumscribed by the "Cosmetics, Toiletries and Fragrances Association (CTFA)", Vol. 1. p. 757 (1995) by the chemical formula

Me₃SiO-[Si(OSiMe₃)(Ph)O]ₙ-SiMe₃.

Such products are marketed, for example, by Dow Corning Corporation and by the General Electric Company.

However, analyses of these products reveal the presence of considerable proportions of methoxy groups. Methoxy groups in silicones are chemically unstable, tend toward hydrolysis and methanol formation and are thus undesired in cosmetic products.

United States Patent 5,679,822 describes the preparation of phenylsilsesquioxane liquids, which are substantially free from alkoxysilanes, chlorosilanes, silanols, hexamethyldisiloxanes, organic compounds and inorganic compounds.

### DESCRIPTION OF THE PRESENT INVENTION

Surprisingly, it has now been found that the phenylsilsesquioxane liquids prepared in accordance with the above-cited UNITED states Patent 5,679,822 are very highly suitable for the formulation of cosmetic and pharmaceutical compositions.

The phenylsilsesquioxane liquids described in United States Patent 5,679,822 surprisingly have improved spreadability and dispersibility and very good compatibility with organic constituents, in particular with oils, waxes and aromatic constituents. In addition, they are characterized by very good skin sensory properties and have a lustrous and care effect on the hair. Moreover, they have a good gliding action and carrier action. The phenylsilsesquioxane liquids prepared in accordance with the method described in United States Patent 5,679,822 can be used either as spreading agent and dispersant, bodying agent, glidant, conditioner, repellent and luster agent and are suitable in an excellent manner for the preparation of a large number of cosmetic and pharmaceutical compositions from the area of cleaning, care, healing, deodorizing, decorative and antiperspirant products.

The invention provides for the use of phenylsilsesquioxane liquids of the general composition

Me₃SiO-[Si(OSiMe₃)(Ph)O]ₓ-SiMe₃

wherein Me is methyl, Ph is phenyl and x is an integer between 1 and 8, wherein the phenylsilsesquioxanes are substantially free from alkoxysilanes, chlorosilanes, silanols, hexamethyl-disiloxanes, organic compounds and inorganic compounds, for the preparation of cosmetic and pharmaceutical compositions.

Preference is given to phenylsilsesquioxane liquids with a purity equal to or greater than 99.5% by weight, particularly preferably equal to or greater than 99.9% by weight.

Preference is given to compositions with x values of from 1 to 3, particularly preferably x is 1. The phenylsilsesquioxane liquids are preferably prepared in accordance with US 5,679,822, the entire contents of which are hereby expressly incorporated into the present application.

Preferably, the phenylsilsesquioxane liquids are prepared by
a)
   i) hydrolyzing a mixture of pure trimethylchlorosilane and pure phenyltrichlorosilane with distilled water, the amount of water being chosen such that it is sufficient to form an aqueous layer which comprises less than 25% by weight of hydrochloric acid, and where the temperature of the hydrolysis reaction is maintained below 90°C in order to form a silicone intermediate; then removing the residual acid from the silicone intermediate; then azeotropically removing the water from the washed silicone intermediate, in order to convert said intermediate into a dried silicone intermediate; and subsequently trimethylsilylating the silanol groups in the dried silanol intermediate with at least a stoichiometric amount of hexamethyldisiloxane in the presence of an acidic catalyst.

Preferred embodiments of the preparation process are those described in United States Patent 5,679,822.

Advantageously, the preparation method described in United States Patent 5,679,822 permits the preparation of phenylsilsesquioxane liquids with a broad viscosity spectrum. Preference is given to phenylsilsesquioxane liquids with viscosities of from 5 cs to 2000 cs, preferably 10 to 1000 cs, particularly preferably from 25 cs to 500 cs, at a temperature of 25°C.

This wide range of viscosities allows the formulator to influence the rheology of cosmetic products and thus the visual appearance of a cream or lotion. The sensory properties, such as consistency or ability to be distributed, determine the individual profile of a cosmetic product. The effectiveness of active substances (e.g. sunscreen filters) and also the storage stability of the formulation are highly dependent on the rheological properties of the product.

The phenylsesquisiloxane liquids are present in the compositions according to the invention, based on the finished composition, preferably in amounts of from 0.01 to 60% by weight, preferably 0.1 to 50% by weight, particularly preferably 0.5 to 5% by weight.

The cosmetic and pharmaceutical compositions may be rinse-off products, preferably shampoos, shower preparations, shower gels, foam baths, or else leave-on products, preferably skin care compositions, day creams, night creams, care creams, nourishing creams, body lotions, ointments, sunscreens, lip care compositions and deodorants.

The compositions may also be surfactant-free aqueous compositions or emulsions, for example hair cures and hair rinses, hair gels, and also permanent waving compositions, hair-coloring compositions, and decorative cosmetics, for example make-up, eyeshadows, lipsticks, mascara and the like, and also purely lipidic systems, in particular fluids.

The cosmetic and pharmaceutical compositions are usually adjusted to a pH of from 2 to 12, preferably pH 3 to 8.

The cosmetic and pharmaceutical compositions can comprise, as further auxiliaries and additives, all customary anionic, cationic, zwitterionic, nonionic and amphoteric surfactants, oily substances, emulsifiers and coemulsifiers, cationic polymers, film formers, and also further additives customary in cosmetics and pharmacy, such as, for example, superfatting agents, moisture-donating agents, stabilizers, biogenic active ingredients, glycerol, preservatives, pearlizing agents, dyes and fragrances, solvents, hydrotropic agents, opacifiers, thickeners and dispersants, and also protein derivatives, such as gelatin, collagen hydrolyzates, natural- and synthetic-based polypeptides, egg yolk, lecithin, lanolin and lanolin derivatives, fatty alcohols, silicones, deodorizing agents, substances having a keratolytic and keratoplastic action, enzymes and carrier substances, antioxidants, UV light protection filters, pigments and metal oxides, and also antimicrobial agents.

Suitable anionic surfactants are, preferably, (C₁₀-C₂₀)-alkyl and alkylene carboxylates, alkyl ether carboxylates, fatty alcohol sulfates, fatty alcohol ether sulfates, alkylamide sulfates and sulfonates, fatty acid alkylamide polyglycol ether sulfates, alkanesulfonates and hydroxyalkanesulfonates, olefinsulfonates, acyl esters of isethionates, α-sulfo fatty acid esters, alkylbenzenesulfonates, alkylphenol glycol ether sulfonates, sulfosuccinates, sulfosuccinic monoesters and diesters, fatty alcohol ether phosphates, protein-fatty acid condensation products, alkyl monoglyceride sulfates and sulfonates, alkyl glyceride ether sulfonates, fatty acid methyltaurides, fatty acid sarcosinates, sulforicinoleates, acyl glutamates. These compounds and mixtures thereof are used in the form of their water-soluble or water-dispersible salts, for example the sodium, potassium, magnesium, ammonium, mono-, di- and triethanolammonium and analogous alkylammonium salts.

The proportion by weight of the anionic surfactants in the compositions according to the invention, based on the finished compositions, is preferably in the range from 2 to 30% by weight, particularly preferably 5 to 25% by weight, especially preferably 12 to 22% by weight.

Preferred cationic surfactants are, for example, quaternary ammonium salts, such as di(C₁₀-C₂₄)alkyldimethylammonium chloride or bromide, preferably di(C₁₂-C₁₈)-alkyldimethylammonium chloride or bromide; (C₁₀-C₂₄)alkyldimethyl-ethylammonium chloride or bromide; (C₁₀-C₂₄)alkyltrimethylammonium chloride or bromide, preferably cetyltrimethylammonium chloride or bromide and (C₂₀-C₂₂)alkyltrimethylammonium chloride or bromide; (C₁₀-C₂₄)alkyldimethylbenzylammonium chloride or bromide, preferably (C₁₂-C₁₈)alkyldimethylbenzylammonium chloride; N-(C₁₀-C₁₈)alkylpyridinium chloride or bromide, preferably N-(C₁₂-C₁₆)alkylpyridinium chloride or bromide; N-(C₁₀-C₁₈)alkylisoquinolinium chloride, bromide or monoalkyl sulfate; N-(C₁₂-C₁₈)alkylpolyoylaminoformylmethylpyridinium chloride; N-(C₁₂-C₁₈)alkyl-N-methylmorpholinium chloride, bromide or monoalkyl sulfate; N-(C₁₂-C₁₈)alkyl-N-ethylmorpholinium chloride, bromide or monoalkyl sulfate; (C₁₆-C₁₈)alkylpentaoxethylammonium chloride; diisobutylphenoxyethoxyethyldimethylbenzylammonium chloride; salts of N,N-diethylaminoethylstearylamide and -oleylamide with hydrochloric acid, acetic acid, lactic acid, citric acid, phosphoric acid; N-acylaminoethyl-N,N-diethyl-N-methylammonium chloride, bromide or monoalkyl sulfate and N-acylaminoethyl-N,N-diethyl-N-benzylammonium chloride, bromide or monoalkyl sulfate.

The proportion by weight of cationic surfactants in the compositions is preferably in the range from 1 to 10% by weight, particularly preferably 2 to 7% by weight, especially preferably 3 to 5% by weight.

Preferred nonionic surfactants are fatty alcohol ethoxylates (alkylpolyethylene glycols); alkylphenol polyethylene glycols; alkyl mercaptan polyethylene glycols; fatty amine ethoxylates (alkylaminopolyethylene glycols); fatty acid ethoxylates (acylpolyethylene glycols); polypropylene glycol ethoxylates (Pluronics®); fatty acid alkylolamides; (fatty acid amide polyethylene glycols); N-alkyl-, N-alkoxypolyhydroxy fatty acid amides; sucrose esters; sorbitol esters and of polyglycol ethers.

The proportion by weight of nonionic surfactants in the compositions is preferably in the range from 1 to 20% by weight, particularly preferably 2 to 10% by weight, especially preferably 3 to 7% by weight.

Preferred amphoteric surfactants are N-(C₁₂-C₁₈)alkyl-β-aminopropionates and N-(C₁₂-C₁₈)alkyl-β-iminodipropionates as alkali metal and mono-, di- and trialkylammonium salts; N-acylaminoalkyl-N,N-dimethylacetobetaines, preferably N-(C₈-C₁₈)acylaminopropyl-N,N-dimethylacetobetaine; (C₁₂-C₁₈)alkyldimethylsulfopropylbetaines; amphoteric surfactants based on imidazoline (trade names Miranol®, Steinapon®), preferably the sodium salt of 1-(β-carboxymethyloxyethyl)-1-(carboxymethyl)-2-Iaurylimidazolinium; amine oxides, preferably (C₁₂-C₁₈)alkyldimethylamine oxides; fatty acid amidoalkyldimethylamine oxides.

The proportion by weight of amphoteric surfactants in the compositions is preferably in the range from 0.5 to 20% by weight, particularly preferably 1 to 10% by weight.

Furthermore, foam-boosting cosurfactants from the group of alkylbetaines, alkylamidobetaines, aminopropionates, aminoglycinates, imidazoliniumbetaines, sulfobetaines, amine oxides, fatty acid alkanolamides and polyhydroxyamides can be used in compositions.

Particularly preferred surfactants are lauryl sulfate, laureth sulfate, cocamidopropylbetaine, sodium cocoyl glutamate and/or lauroamphoacetate.

In a preferred embodiment of the invention, the phenylsesquisiloxane liquids are used for the preparation of leave-on products, in particular emulsions.

The emulsions may either be water-in-oil emulsions or oil-in-water emulsions, microemulsions and multiple emulsions. The emulsions can be prepared in a known manner, i.e. for example by hot, hot/cold or PIT emulsification.

The nonaqueous proportion of the emulsions, which is comprised largely of the emulsifier, the thickener and the oily substance, is preferably 5 to 95% by weight, preferably 15 to 75% by weight. The water proportion of the emulsions is thus preferably 5 to 95% by weight, preferably 25 to 85% by weight, depending on whether the intention is to prepare, for example, lotions with a relatively low viscosity, or, for example, creams and ointments with a high viscosity.

Oily substances are to be understood as meaning any fatty substance which is liquid at room temperature (25°C). The fatty phase can comprise one or more oils which are preferably chosen from the following oils:
silicone oils, volatile or nonvolatile, linear, branched or cyclic, for example cyclomethicones, possibly organically modified; and
silicone resins and gums which are solid or liquid at room temperature; and
mineral oils, such as paraffin or vaseline oil; and
oils of animal origin, such as perhydrosqualene and lanolin; and
oils of vegetable origin, e.g. liquid triglycerides, such as sunflower oil, corn oil, soybean oil, rice oil, jojoba oil, babussu oil, pumpkin oil, grapeseed oil, sesame oil, walnut oil, apricot oil, macadamia oil, avocado oil, sweet almond oil, lady's smock oil, castor oil, triglycerides of caprylic/capric acids, olive oil, groundnut oil, rapeseed oil and coconut oil.

Also suitable as oils are synthetic oils, such as purcellin oil, isoparaffins, linear and/or branched fatty alcohols and fatty acid esters, preferably Guerbet alcohols having 6 to 18, preferably 8 to 10, carbon atoms; esters of linear (C₆-C₁₃)-fatty acids with linear (C₆-C₂₀)-fatty alcohols; esters of branched (C₆-C₁₃)-carboxylic acids with linear (C₆-C₂₀)-fatty alcohols, esters of linear (C₆-C₁₈)-fatty acids with branched alcohols, in particular 2-ethylhexanol; esters of linear and/or branched fatty acids with polyhydric alcohols (such as, for example, dimerdiol or trimerdiol) and/or Guerbet alcohols; triglycerides based on (C₆-C₁₀)-fatty acids.

Also suitable are esters such as dioctyl adipate, diisopropyl dimer dilinoleate, propylene glycol/dicaprylate or waxes, such as beeswax, paraffin wax or microcrystalline waxes, optionally in combination with hydrophilic waxes, such as, for example, cetylstearyl alcohol. Also suitable are fluorinated and perfluorinated oils, fluorinated silicone oils or mixtures thereof.

Suitable nonionogenic coemulsifiers are preferably addition products of up to 30 mol of ethylene oxide and/or up to 5 mol of propylene oxide onto linear fatty alcohols having 8 to 22 carbon atoms, onto fatty acids having 12 to 22 carbon atoms, onto alkylphenols having 8 to 15 carbon atoms in the alkyl group and onto sorbitan or sorbitol esters; (C₁₂-C₁₈)-fatty acid mono- and diesters of addition products of up to 30 mol of ethylene oxide onto glycerol; glycerol mono- and diesters and sorbitan mono- and diesters of saturated and unsaturated fatty acids having 6 to 22 carbon atoms and optionally ethylene oxide addition products thereof; addition products of from 15 to 60 mol of ethylene oxide onto castor oil and/or hydrogenated castor oil; polyol and, in particular, polyglycerol esters, such as, for example, polyglycerol polyricinoleate and polyglycerol poly-12-hydroxystearate; mixtures of the abovementioned compounds.

Examples of suitable ionogenic coemulsifers are anionic emulsifiers, such as mono-, di- or triphosphoric esters, but also cationic emulsifiers, such as mono-, di- and trialkyl quats and polymeric derivatives thereof.

Suitable cationic polymers are, preferably, those known under the INCI name "Polyquaternium", in particular Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, and Polyquaternium 37&mineral oil&PPG trideceth (Salcare® SC95), PVP dimethylaminoethyl methacrylate copolymer, guar hydroxypropyltriammonium chloride, and calcium alginate and ammonium alginate. It is also possible to use cationic cellulose derivatives; cationic starch; copolymers of diallylammonium salts and acrylamides; quaternized vinylpyrrolidone/vinyl-imidazole polymers; condensation products of polyglycols and amines; quaternized collagen polypeptides; quaternized wheat polypeptides; polyethyleneimines; cationic silicone polymers, such as, for example, amidomethicones; copolymers of adipic acid and dimethylaminohydroxypropyldiethylenetriamine; polyamino-polyamide and cationic chitin derivatives, such as, for example, chitosan. Suitable silicone compounds are, for example, dimethylpolysiloxane, methylphenylpolysiloxanes, cyclic silicones and amino-, fatty-acid-, alcohol-, polyether-, epoxy-, fluorine- and/or alkyl-modified silicone compounds, and polyalkylsiloxanes, polyalkylarylsiloxanes, polyether siloxane copolymers, as described in US 5104 645 and the publications cited therein, which may either be liquid or else resin-like at room temperature.

Depending on the intended use, suitable film formers are water-soluble polyurethanes, for example C₁₀-polycarbamyl polyglyceryl ester, polyvinyl alcohol, polyvinylpyrrolidone, -copolymers, for example vinylpyrrolidone/vinylacetate copolymer, water-soluble acrylic acid polymers/copolymers and esters or salts thereof, for example partial ester copolymers of acrylic/methacrylic acid and polyethylene glycol ethers of fatty alcohols, such as acrylate/steareth-20-methacrylate copolymer, water-soluble cellulose, for example hydroxymethylcellulose, hydroxyethylcellulose, hydroxypropylcellulose, water-soluble quaterniums, polyquaterniums, carbocyvinyl polymers, such as carbomers and salts thereof, polysaccharides, for example polydextrose and glucan.

Superfatting agents which may be used are substances such as, for example, polyethoxylated lanolin derivatives, lecithin derivatives, polyol fatty acid esters, monoglycerides and fatty acid alkanolamides, the latter also serving as foam stabilizers. Examples of moisture-donating substances, which are available, are isopropyl palmitate, glycerol and/or sorbitol.

Stabilizers, which can be used, are metal salts of fatty acids, such as, for example, magnesium stearate, aluminum stearate and/or zinc stearate. Biogenic active ingredients are to be understood as meaning, for example, plant extracts and vitamin complexes.

In addition, the compositions can comprise organic solvents. In principle, suitable organic solvents are all mono- or polyhydric alcohols. Preference is given to using alcohols having 1 to 4 carbon atoms, such as ethanol, propanol, isopropanol, n-butanol, i-butanol, t-butanol, glycerol and mixtures of said alcohols. Further preferred alcohols are polyethylene glycols with a relative molecular mass below 2000. In particular, a use of polyethylene glycol with a relative molecular mass between 200 and 600 and in amounts up to 45% by weight, and of polyethylene glycol with a relative molecular mass between 400 and 600 in amounts of from 5 to 25% by weight is preferred. Further suitable solvents are, for example, triacetin (glycerol triacetate) and 1-methoxy-2-propanol.

The compositions can be mixed with conventional ceramides, pseudoceramides, fatty acid N-alkylpolyhydroxyalkylamides, cholesterol, cholesterol fatty acid esters, fatty acids, triglycerides, cerebrosides, phospholipids and similar substances as care additive.

In order to adjust the rheological properties of aqueous or solvent-containing emulsions or suspensions, a large number of different systems are given in the specialist literature. Known examples are cellulose ethers and other cellulose derivatives (e.g. carboxymethylcellulose, hydroxyethylcellulose), gelatin, starch and starch derivatives, sodium alginate, fatty acid polyethylene glycol esters, agar agar, tragacanth or dextrins. The synthetic polymers used are various materials, such as, for example, polyvinyl alcohols, polyacrylamides, polyvinylamides, polysulfonic acids, polyacrylic acid, polyacrylates, polyvinylpyrrolidone, polyvinyl methyl ether, polyethylene oxides, copolymers of maleic anhydride and vinyl methyl ether, and various mixtures and copolymers of the abovementioned compounds, including their various salts and esters. These polymers can be crosslinked or uncrosslinked, as desired.

### Examples of suitable UV filters are 4-aminobenzoic acid;

Pigments/micropigments which may be used are, for example, microfine titanium dioxide, mica-titanium oxide, iron oxides, mica-iron oxide, zinc oxide, silicon oxides, ultramarine blue or chromium oxides.

Examples of suitable antioxidants are superoxide dismutase, tocopherol (vitamin E) and ascorbic acid (vitamin C).

Examples of suitable preservatives are phenoxyethanol, parabens, pentanediol or sorbic acid.

Dyes which can be used are the substances approved and suitable for cosmetic purposes.

Suitable fungicidal active ingredients are, preferably, ketoconazole, oxiconazole, bifonazole, butoconazole, cloconazole, clotrimazole, econazole, enilconazole, fenticonazole, isoconazole, miconazole, sulconazole, tioconazole, fluconazole, itraconazole, terconazole, naftifine and terbinafine, Znpyrethione and octopyrox.

Advantageously, the emulsions prepared according to the invention are extremely creamy and ointment-like and can be applied very finely. The emulsions give the skin an exceptionally good feel, and when applied, the emulsions give a feeling of freshness and of comfort, and they have a nourishing effect at the same time. In addition, the compositions are soft and luxurious and in no way sticky.

The invention also provides for the use of phenylsilsesquioxane liquids of the formula (I)

Me₃SiO-[Si(OSiMe₃)(Ph)O]ₓ-SiMe₃ (I)

wherein Me is methyl, Ph is phenyl and x is an integer between 1 and 8,
wherein the phenylsilsesquioxanes are substantially free from alkoxysilanes, chlorosilanes, silanols, hexamethyldisiloxanes, organic compounds and inorganic compounds, for the preparation of cosmetic and pharmaceutical compositions.

The phenylsilsesquioxane liquids are preferably used as spreading agent, dispersant, bodying agent, carrier, glidant, conditioner, repellent and luster agent in cosmetic and pharmaceutical compositions.

The phenylsilsesquioxane liquids used are preferably prepared in accordance with the process(es) described in United States Patent 5,679,822.

### EXAMPLES

The examples and uses below serve to illustrate the invention in more detail, without, however, limiting it thereto. All percentages are in % by weight.

### Example 1: 2 in 1 Conditioning Shampoo

| Ingredients | % w/w | Trade name | Supplier |
|---|---|---|---|
| Phase A | | | |
| Deionized Water | Q.S. | N/A | N/A |
| Sodium Laureth (2) sulfate | 49.80 | Rhodapex ES-2 | Rhodia |
| Sodium Lauryl Sulfate | 13.60 | Rhodapon SB-8208/S | Rhodia |
| Cocamidopropyl | 8.10 | Crosultaine C-50 | Croda Hydroxysultaine |
| Cocamide MEA | 5.10 | Colamid CMA | Colonial |
| Polyquaternium-11 | 1.60 | Gafquat 734 | ISP |
| Steareth-2 | 0.90 | Brij 721 | Uniqema |
| Steareth-21 | 0.10 | Brij 72 | Uniqema |
| Phenyl Trimethicone | 1.00 | SilCare 15M40 | Clariant |
| Panthenol | 1.00 | Panthenol | Jeen Int |
| Methylparaben | 0.20 | Nipagin M | NIPA |
| Propylparaben | 0.10 | Nipasol M | NIPA |
| Disodium EDTA | 0.10 | Dissolvine Na2 | Akzo |

| Phase B | | | |
|---|---|---|---|
| Fragrance | 0.30 | | |

Manufacturing Procedure:
Heat water of Phase A to 50 - 55°C
While slowly mixing, add the ingredients of Phase A, one by one
Cool Phase A to 30 - 35°C
Add Phase B (fragrance). Mix well until clear.

### Example 2: Anhydrous Stick

| Ingredients | %w/w | Trade name | Supplier |
|---|---|---|---|
| Phase A | | | |
| Stearyl alcohol | 22.30 | Codacol S-95 | Croda |
| C₂₄-₂₈-Alkyl dimethicone | 10.00 | SilCare 41M80 | Clariant |
| Tribehenin and | 7.50 | Syncrowax HRS-C | Croda |
| Calcium Behenate | | | |
| PPG-2 Myristyl | 5.00 | Crodamol PMP | Croda |
| Ether Propionate | | | |
| Paraffin | 3.00 | Paraffin 201 | Koster Keunen |

| Phase B | | | |
|---|---|---|---|
| Phenyl Trimethicone | 52.00 | SilCare 15M60 | Clariant |
| Fragrance | 0.20 | | |

Manufacturing Procedure:
Heat Phase A to 90 - 95°C. Mix well.
Initiate slow cooling. Mix and add Phase B.
Mix and cool to 40°C. Add fragrance.

### Example 3: Antifrizz Hair Gel

| Ingredients | %w/w | Trade name | Supplier |
|---|---|---|---|
| Phase A | | | |
| Dipropylene glycol | 40.00 | Dipropylene glycol | Arco |
| Distilled water | 25.30 | N/A | N/A |

| Phase B | | | |
|---|---|---|---|
| Phenyl Trimethicone LV | 8.00 | SilCare 15M60 | Clariant |
| Phenyl Trimethicone SV | 7.00 | SilCare 31M50 | Clariant |

| Phase C | | | |
|---|---|---|---|
| Dimethicone | 1.60 | SE-30 | GE |
| Phenyl Trimethicone HV | 14.40 | SilCare 15M30 | Clariant |

| Phase D | | | |
|---|---|---|---|
| Polyacrylamide, Laureth-7 and C₁₃₋₁₄ Isoparaffin | 2.50 | Sepigel 305 | Seppic |

| Phase E | | | |
|---|---|---|---|
| lodopropynyl Butylcarbamate | 1.00 | Jeecide IPBC, 10% | Jeen Int |

Manufacturing procedure:
Combine ingredients of Phase A
Combine ingredients of Phase B
Solubilize Dimethicone in Phenyl Trimethicone
Mix Phases A, B and C
With vigorous mixing, add Sepigel 305 and mix until the product is uniform and lump-free.
Add ingredients of Phase E with mixing within 2-3 minutes.

### Example 4: Baby Protectant Cream

| Ingredients | %w/w | Trade name | Supplier |
|---|---|---|---|
| Phase A | | | |
| Deionized water | Q.S. | N/A | N/A |
| Sodium Chloride | 0.60 | Sodium Chloride | various |
| Phenoxyethanol and | 0.25 | Phenonip | NIPA |
| Methylparaben and Ethylparaben and Butylparaben and Propylparaben and Isopropylparaben | | | |

| Part B | | | |
|---|---|---|---|
| Phenyl Trimethicone | 5.00 | SilCare 15M60 | Clariant |
| White petrolatum USP | 4.50 | Ultrapure SC | Ultra |
| Stearoxytrimethylsilane | 3.00 | SilCare 1M71 | Clariant |
| Stearyl Dimethicone | 1.50 | SilCare 41 M65 | Clariant |
| Ethylhexyl Stearate | 5.00 | Tegosoft OS | Goldschmidt |
| Light Mineral Oil NF | 4.00 | Ultrasol 70 | Ultra |
| Polyglyceryl-6 Isostearate | 0.50 | Plurol Isostearique | Gattefosse |
| Hydrogenated Castor Oil | 0.80 | Cutina HR | Henkel |
| White Beeswax NF | 1.20 | Beeswax | Hansotech |

| Part C | | | |
|---|---|---|---|
| Fragrance | 0.25 | | |

Manufacturing Procedure:
Heat Phase A to 50-55°C
Heat Phase B to 80-85°C
Emulsify at high speed, while slowly adding Phase A to Phase B. Cool to 50°C.
Mix and cool below 35°C. Homogenize to obtain a smooth cream.
While slowly mixing add fragrance. Mix for 3-5 minutes.

### Example 5: Moisturizer

| Ingredients | %w/w | Trade name | Supplier |
|---|---|---|---|
| Phase A | | | |
| Cetyl Dimethicone | 2.00 | Abil EM 90 | Goldschmidt |
| Copolyol | | | |
| Microcrystalline Wax | 1.40 | SP-89 | Strahl&Pitsch |
| Hydrogenated Castor Oil | 1.00 | Hansonwax JH80 | Hanson |
| Caprylic/Capric | 8.00 | Labrafac WL1349 | Gattefosse |
| Triglyceride | | | |
| Octyl Palmitate | 4.00 | Trivent OP | Trivent |
| Decyl Oleate | 4.00 | Cetiol V | Henkel |

| Phase B | | | |
|---|---|---|---|
| Phenyl Trimethicone | 10.00 | SilCare 15M30 | Clariant |

| Phase C | | | |
|---|---|---|---|
| Deionized Water | 60.90 | N/A | N/A |
| Sodium Chloride | 0.50 | N/A | N/A |
| Urea | 2.00 | Urea | E M Ind |
| SD Alcohol 40B | 5.00 | Alcohol | Local |

| Phase D | | | |
|---|---|---|---|
| lodopropynyl Butylcarbamate | 1.00 | Nipaguard IPF | Clariant |

Manufacturing procedure:
Heat Phase A to 50-55°C
Mix and cool to 45°C
Add SilCare 15M30. Mix well.
Separately, mix Phase C ingredients. Dissolve solids.
While mixing with a paddle impeller at moderate speed, slowly
add Phase C to Phase (A+B) to form a cream. Homogenize.
While slowly mixing, add preservative and fragrance.

### Example 6: Sunscreen Cream

| Ingredients | %w/w | Trade name | Supplier |
|---|---|---|---|
| Phase A | | | |
| Deionized Water | 60.64 | N/A | N/A |
| Glycerin, USP | 5.00 | Glycerin | Local |
| Carbomer | 0.40 | Carbopol Ultrez10 | BFGoodrich Disodium |
| EDTA | 0.50 | Dissolvine NA2 | AKZO |

| Phase B | | | |
|---|---|---|---|
| Sodium Hydroxide, 20% | 0.40 | Sodium Hydroxide | JT Baker |

| Phase C | | | |
|---|---|---|---|
| Cetyl Alcohol, 2.00 Emulsire 61 WL Gattefosse and Ceteth-20 and Steareth-20 | | | |
| Glyceryl Stearate and PEG-100 Stearate | 5.00 | Lipomulse 165 | Lipo |
| Cetyl Alcohol, NF | 0.50 | Crodacol C-95 | Croda |
| Stearyl Alcohol, NF | 1.50 | Crodacol S-95 | Croda |
| Phenyl Trimethicone | 7.50 | SilCare 15M40 | Clariant |
| Isostearyl Isostearate | 4.00 | Isos. D'isostearyle | Gattefosse |
| Octyl Methoxycinnamate | 7.50 | Parsol MCX | Roche |
| Butyl Methoxydibenzoylmethane | 1.50 | Parsol 1789 | Roche |
| Butylated hydroxytoluene | 0.01 | BHT | Nipa |

| Phase D | | | |
|---|---|---|---|
| Hexyl Methicone | 2.25 | Silcare 41M10 | Clariant |
| Dimethicone | 0.25 | SE-30 | Gen. Electric |

| Phase E | | | |
|---|---|---|---|
| Iodopropynylbutylcarbamate | 1.00 | Jeecide IPBC, 10% | Jeen Int |
| Fragrance | 0.50 | | |

Manufacturing procedure:
Disperse carbomer in water. Heat dispersion to 75-80°C. Add glycerin and disodium EDTA.
After dissolution of the EDTA continue mixing and add sodium hydroxide (20% solution).
Heat separately Phase C to 75-80°C. Then add Phases A+B.
Continue mixing and cool down to 40-45°C.
Solubilize dimethicone in Hexyl Methicone. Then add to batch.
Continue mixing and cool to 35°C. Add Phase E ingredients.
Mix to uniformity.

### Example 7: Waterproof Sunscreen Spray Mist

| Ingredients | %w/w | Trade name | Supplier |
|---|---|---|---|
| Phase A | | | |
| Anhydrous Ethanol SDA 3A | 64.20 | SDA 3A | Various |
| Deionized Water | Q.S. | N/A | N/A |
| Acrylates/Octylacrylamide | 1.00 | Dermacryl 79 LT | Natl. Starch |

| Part B | | | |
|---|---|---|---|
| Octyl Methoxycinnamate | 7.50 | Parsol MCX | Roche |
| Benzophenone-3 | 3.00 | Escalol 567 | ISP |
| Methyl Anthranilate | 3.50 | Dermablock | Alzo |
| Phenylsilsesquioxane | 5.00 | SilCare 15M60 | Clariant |
| C12-15 Alkyl benzoates | 10.00 | Finsolv TN | Finetex |
| Butylated hydroxytoluene | 0.02 | BHT | Nipa |
| Propylparaben | 0.10 | Nipasol | Nipa |
| Vitamin E | 0.25 | Vitamin E | Protameen |

| Phase C | | | |
|---|---|---|---|
| Fragrance | 0.50 | | |

Manufacturing procedure:
Mix water and ethanol. Slowly disperse Dermacryl. Mix to clarity.
2. Add ingredients of Phase B one by one, mix until clear.
3. Add fragrance. Mix until clear.

### Example 8: Antiperspirant Cream

| Ingredients | %w/w | Trade name | Supplier |
|---|---|---|---|
| Phase A | | | |
| Cetyl alcohol | 2.5 | Crodacol C-95 | Croda |
| Stearyl alcohol | 2.5 | Crodacol S-95 | Croda |
| Aluminum Chlorhydrate | 10.0 | Locron P | Clariant |
| Oleth-10 and Oleth-5 | 3.0 | Emulgin M8 | Sidobre Sinnova |

| Phase B | | | |
|---|---|---|---|
| Cyclomethicone | 9.0 | Belsil CM 040 | Wacker |
| Phenyl Trimethicone | 3.0 | SilCare 15M50 | Clariant |
| Deionized Water | 63.4 | N/A | N/A |
| Fragrance | q.s. | | |
| Pigments | q.s. | | |

Manufacturing procedure:
Mix A and heat to 70°C
Heat water to 70°C and add to A
Add silicones
Add fragrance and pigments

## Claims

1. The use of phenylsilsesquioxane liquids of the general composition
Me₃SiO-[Si(OSiMe₃)(Ph)O]ₓ-SiMe₃
wherein Me is methyl, Ph is phenyl and x is an integer between 1 and 8, and
wherein the phenylsilsesquioxanes are substantially free from alkoxysilanes, chlorosilanes, silanols, hexamethyldisiloxanes, organic compounds and inorganic compounds, for the preparation of cosmetic and pharmaceutical compositions.

2. The use as claimed in claim 1, wherein the phenylsilsesquioxane liquids have a purity equal to or greater than 99.5% by weight, preferably equal to or greater than 99.9% by weight.

3. The use as claimed in claim 1 or 2, wherein x in the general composition has values from 1 to 3, preferably the value 1.

4. The use as claimed in anyone of claims 1 to 3, wherein the phenylsilsesquioxane liquids have viscosities of from 5 cs to 2000 cs, preferably 10 to 1000 cs, particularly preferably 25 cs to 500 cs, at a temperature of 25°C.

5. The use as claimed in anyone of claims 1 to 4, wherein the cosmetic and pharmaceutical compositions are rinse-off or leave-on products.

6. The use of phenylsilsesquioxane liquids of the general composition
Me₃SiO-[Si(OSiMe₃)(Ph)O]ₓ-SiMe₃
wherein Me is methyl, Ph is phenyl and x is an integer between 1 and 8, and
wherein the phenylsilsesquioxanes are substantially free from alkoxysilanes, chlorosilanes, silanols, hexamethyldisiloxanes, organic compounds and inorganic compounds, as spreading agent, dispersant, bodying agent, carrier, glidant, conditioner, repellent and luster agent in cosmetic and pharmaceutical compositions.

7. The use as claimed in anyone of claims 1 to 6, wherein the phenylsilsesquioxane liquids are prepared by
a)
i) hydrolyzing a mixture of pure trimethylchlorosilane and pure phenyltrichlorosilane with distilled water, the amount of water being chosen such that it is sufficient to form an aqueous layer which comprises less than 25% by weight of hydrochloric acid, and where the temperature of the hydrolysis reaction is maintained below 90°C in order to form a silicone intermediate; then
ii) removing the residual acid from the silicone intermediate; then
iii) azeotropically removing the water from the washed silicone intermediate, and converting the latter into a dried silicone intermediate; and subsequently
b) trimethylsilylating the silanol groups in the dried silanol intermediate with at least a stoichiometric amount of hexamethyldisiloxane in the presence of an acidic catalyst.
